# EUROPÄISCHE PATENTSCHRIFT

(11) **EP 0 965 591 B1**
(45) Veröffentlichungstag und Bekanntmachung des Hinweises auf die Patenterteilung: **04.09.2002**
(21) Anmeldenummer: 99101810.2
(22) Anmeldetag: 21.06.1991
(51) Int. Cl.: C07D 295/12, A61K 31/445

(54) **Verfahren zur Herstellung von (S)-3-Methyl-1-(2-piperidino-phenyl)-1-butylamin**
Process for the preparation of (S)-3-methyl-1-(2-piperidino-phenyl)-1-butylamine
Procédé pour la préparation de (S)-3-methyl-1-(2-piperidino-phényl-1-butylamine

(43) Veröffentlichungstag der Anmeldung: 22.12.1999
(62) Teilanmeldung aus: 91911959.4
(73) Patentinhaber: Boehringer Ingelheim Pharma KG, 55218 Ingelheim am Rhein (DE)
(72) Erfinder: Grell, Wolfgang Dr. Dipl.-Chem., 88400 Biberach (DE); Greischel, Andreas Dr., 88400 Biberach (DE); Zahn, Gabriele, Frau Dr., 88499 Riedlingen (DE); Mark, Michael Dr., 88400 Biberach (DE); Knorr, Marianne, 55218 Ingelheim/Rhein (DE); Eckhard, Rupprecht, 2353 Guntramsdorf (AT); Müller, Ulrich Dr., 88441 Biberach-Stafflangen (DE)
(74) Vertreter: Laudien, Dieter, Dr.

(56) Entgegenhaltungen:
- EP-A- 0 128 482
- EP-A- 0 147 850
- EP-A- 0 207 331
- GREISCHEL, ANDREAS ET AL: "Quantitation of the new hypoglycemic agent AG-EE 388 ZW in human plasma by automated high-performance liquid chromatography with electrochemical detection" J. CHROMATOGR. (1991), 568(1), 246-52 , 1991, XP002117138
- DATABASE WPI Derwent Publications Ltd., London, GB; AN 1989-195144 XP002117139 "Optical resolution of D,L carnitine nitrile chloride - using N-acetyl- D or L- glutamic acid as resolving agent" & JP 01 131143 A (EARTH SEIYAKU KK)

## Beschreibung

In der EP-B-0,147,850 wird unter anderem das Racemat von 2-Ethoxy-4-[N-[1-(2-piperidino-phenyl)-3-methyl-1-butyl]aminocarbonylmethyl]benzoesäure (Code-Nr.: AG-EE 388 ZW) der Formel 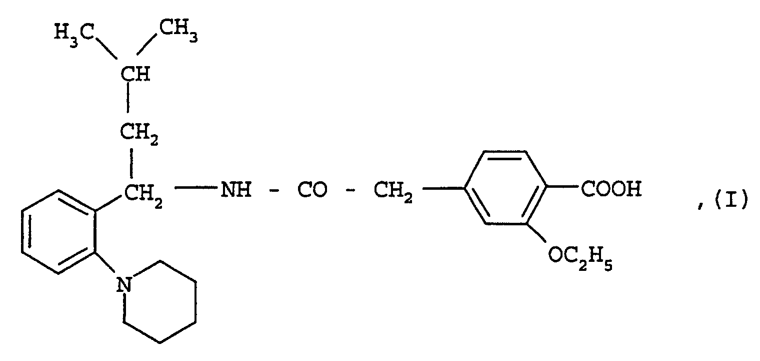 und in der EP-B-0,207,331 werden zwei weitere polymorphe Formen dieser Verbindung beschrieben. Diese Verbindung und deren physiologisch verträgliche Salze weisen wertvolle pharmakologische Eigenschaften auf, nämlich eine Wirkung auf den Intermediärstoffwechsel, insbesondere jedoch eine blutzuckersenkende Wirkung.

Die beiden Enantiomeren dieser Verbindung, nämlich (S)(+)-2-Ethoxy-4-[N-[1-(2-piperidino-phenyl)-3-methyl-1-butyl]-aminocarbonylmethyl]benzoesäure (Code-Nr.: AG-EE 623 ZW) und (R) (-)-2-Ethoxy-4-[N-[1-(2-piperidino-phenyl)-3-methyl-1-butyl]aminocarbonylmethyl]benzoesäure (Code-Nr.: AG-EE 624 ZW), wurden auf ihre blutzuckersenkende Wirkung an weiblichen Ratten geprüft.

Es wurde gefunden, daß das (S)-Enantiomere (AG-EE 623 ZW) das wirksame Enantiomere ist, und daß seine Wirkung länger als 6 Stunden an der Ratte anhält.

Auf Grund dieser Ergebnisse an der Ratte erscheint für den Menschen die ausschließliche Verwendung von AG-EE 623 ZW geboten, wodurch die Dosis im Vergleich zur AG-EE 388 ZW-Dosis um 50 % reduziert werden kann. Dies und eine relativ lange Wirkungsdauer konnten am Menschen bestätigt werden. Darüber hinaus wurde jedoch bei den Human-Studien gefunden, daß AG-EE 623 ZW überraschende pharmakokinetische Eigenschaften aufweist, die auf Grund der AG-EE 388 ZW-Daten nicht vorhersehbar waren. AG-EE 623 ZW zeigt somit therapeutische Vorteile gegenüber dem Racemat AG-EE 388 ZW.

Ein wertvolles Zwischenprodukt für die Herstellung von (S)(+)-2-Äthoxy-4-[N-[1-(2-piperidino-phenyl)-3-methyl-1-butyl]aminocarbonylmethyl]benzoesäure ist die Verbindung (S)-1-(2-Piperidino-phenyl)-3-methyl-l-butylamin.

Gegenstand der vorliegenden Erfindung ist somit ein Verfahren zur Herstellung von (S)-1-(2-Piperidino-phenyl)-3-methyl-1-butylamin durch Racematspaltung mittels fraktionierter Kristallisation der diastereomeren Salze mit N-Acetyl-L-glutaminsäure, und erforderlichenfalls Umkristallisation sowie anschließender Zerlegung der Salze.

Ein weiterer Gegenstand der Erfindung ist die Verwendung des so erhaltenen enantiomerenreinen (S)-1-(2-Piperidino-phenyl)-3-methyl-1-butylamins zur Herstellung von (S)-(+)-2-Äthoxy-4-[N-[1-(2-piperidino-phenyl)-3-methyl-1-butyl]aminocarbonylmethyl]benzoesäure, indem das enantiomerenreine (S)-1-(2-Piperidino-phenyl)-3-methyl-1-butylamin
mit einer Carbonsäure der allgemeinen Formel 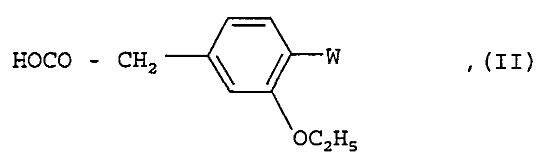 in der
W eine Carboxygruppe oder eine durch einen Schutzrest geschützte Carboxygruppe darstellt,
oder mit deren gegebenenfalls im Reaktionsgemisch hergestellten reaktionsfähigen Derivaten und erforderlichenfalls anschließende Abspaltung eines Schutzrestes.

Als reaktionsfähige Derivate einer Verbindung der allgemeinen Formel II kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.

Die Umsetzung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Chlorameisensäureisobutylester, Thionylchlorid, Phosphortrichlorid, Phosphorpentoxid, N,N'-Dicyclohexylcarbodiimid, N, N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Erforderlichenfalls wird die anschließende Abspaltung eines Schutzrestes vorzugsweise hydrolytisch durchgeführt, zweckmäßigerweise entweder in Gegenwart einer Säure wie Salzsäure, Schwefelsäure, Phosphorsäure, Trifluoressigsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches.

Ein als Schutzrest verwendeter tert.Butylrest kann auch thermisch gegebenenfalls in einem inerten Lösungsmittel wie Methylenchlorid, Chloroform, Benzol, Toluol, Tetrahydrofuran, Dioxan oder Eisessig und vorzugsweise in Gegenwart einer starken Säure wie Trifluoressigsäure, Bromwasserstoff, p-Toluolsulfonsäure, Schwefelsäure, Phosphorsäure oder Polyphosphorsäure abgespalten werden.

Desweiteren kann ein als Schutzrest verwendeter Benzylrest auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid abgespalten werden.

Die nachfolgenden Beispiele sollen die Erfindung näher erläutern:

### Beispiel 1

### (S)-1-(2-Piperidino-phenyl)-3-methyl-1-butylamin

Eine gerührte Lösung von 122 g (0,495 Mol) racemischem 1-(2-Piperidino-phenyl)-3-methyl-1-butylamin in 1000 ml Aceton wird mit 93,7 g (0,495 Mol) N-Acetyl-L-glutaminsäure versetzt. Man erhitzt auf dem Dampfbad unter Rückfluß und gibt portionsweise Methanol (insgesamt ca. 80 ml) zu, bis eine klare Lösung erreicht ist. Nach Abkühlen und Stehenlassen bei Raumtemperatur über Nacht saugt man die erhaltenen Kristalle ab, wäscht diese zweimal mit je 200 ml -15°C kaltem Aceton und trocknet diese. Das erhaltene Produkt [98,9 g; Schmelzpunkt: 163-166°C; [a]²⁰_{D} = + 0,286° (c = 1 in Methanol)] kristallisiert man aus 1000 ml Aceton unter Zusatz von 200 ml Methanol um, wobei man das (S)-1-(2-Piperidino-phenyl)-3-methyl-1-butylamin als N-Acetyl-L-glutaminsäure-Additionssalz erhält.
Ausbeute: 65,1 g (60,4 % der Theorie),
Schmelzpunkt: 168-171°C

| | | | |
|---|---|---|---|
| Ber. | C 63,42 | H 8,56 | N 9,65 |
| Gef. | 63,64 | 8,86 | 9,60 |

[a]²⁰_{D} = + 0,357° (c = 1 in Methanol)

Das freie Amin erhält man als Öl durch Freisetzen beispielsweise mit einer Natriumhydroxid- oder Ammoniak-Lösung, Extraktion beispielsweise mit Toluol, Äther, Äthylacetat oder Methylenchlorid sowie Trocknen, Filtrieren und Eindampfen des Extraktes im Vakuum.

Die (S)-Konfiguration des Amins wurde wie folgt bewiesen: Umsetzung des Amins mit (S')-1-Phenäthylisocyanat in Äther zum entsprechenden Harnstoff-Derivat [Schmelzpunkt: 183-184°C; [a]²⁰_{D} = - 2,25° (c = 1 in Methanol)], Züchtung von Kristallen aus Äthanol/Wasser (8/1) und anschließende Röntgenstruktur-Analyse, die (S,S')-Konfiguration für das Harnstoff-Derivat und somit (S)-Konfiguration für das eingesetzte Amin ergab.

Die Enantiomeren-Reinheit wurde wie folgt bestimmt:
1. Acetylierung einer Probe des Amins mit 1,3 Äquivalenten Acetanhydrid in Eisessig bei 20°C über Nacht.
2. Untersuchung des N-Acetyl-Derivates (Schmelzpunkt: 128-132°C) mittels HPLC auf einer Chiral-Phasen-HPLC-Säule der Firma Baker, bei der (S)-N-(3,5-Dinitrobenzoyl)-2-phe nyl-glycin kovalent an Aminopropyl-Kieselgel gebunden ist (Korngröße: 5 mm, kugelförmig, 60 A Porenweite; Säulen länge: 250 mm bei 4,6 mm Innendurchmesser; Fließmittel: n-Hexan/Isopropanol (100/5); Fließgeschwindigkeit: 2 ml/Minute; Temperatur: 20°C; UV-Detektion bei 254 nm.
Gefunden: Peak 1(R): Peak 2(S) = 0,75 % : 99,25 %,
ee (enantiomeric excess) = 98,5 % (S).

Mittels ätherischer Chlorwasserstoff-Lösung kann das (S)-Amin in sein Dihydrochlorid-hydrat übergeführt werden. Schmelzpunkt: 135-145°C (Zers.)

| | | | | |
|---|---|---|---|---|
| Ber. (x H₂O) | C 56,99 | H 8,97 | N 8,31 | Cl 21,02 |
| Gef. | 56,85 | 8,93 | 8,38 | 21,25 |

[a]²⁰_{D} = + 26,1° (c = 1 in Methanol)

### Beispiel 2

### (S)-2-Äthoxy-4-[N-(1-(2-piperidino-phenyl)-3-methyl-1-butyl)-aminocarbonylmethyl]benzoesäure-äthylester

Zu einer Lösung von 0,47 g (1,91 mMol) (S)-3-Methyl-1-(2-piperidino-phenyl)-1-butylamin (ee = 98,5 %) in 5 ml wasserfreiem Acetonitril gibt man nacheinander 0,48 g (1,91 mMol) 3-Äthoxy-4-äthoxycarbonyl-phenylessigsäure, 0,60 g (2,29 mMol) Triphenylphosphin, 0,80 ml (5,73 mMol) Triäthylamin und 0,18 ml (1,91 mMol) Tetrachlorkohlenstoff und rührt 20 Stunden bei Raumtemperatur. Anschließend dampft man im Vakuum ein und verteilt zwischen Äthylacetat und Wasser. Den organischen Extrakt trocknet und filtriert man und dampft ihn im Vakuum ein. Den Eindampfrückstand reinigt man durch Säulenchromatographie an Kieselgel (Toluol/Äthylacetat = 10/1).
Ausbeute: 0,71 g (77,3 % der Theorie),
Schmelzpunkt: 110-112°C

| | | | |
|---|---|---|---|
| Ber. | C 72,47 | H 8,39 | N 5,83 |
| Gef. | 72,29 | 8,42 | 5,80 |

Die Enantiomeren-Reinheit bestimmt man mittels HPLC auf einer Chiral-Phasen-HPLC-Säule der Firma Baker, bei der (S)-N-3,5-Dinitrobenzoyl-leucin kovalent an Aminopropyl-Kieselgel gebunden ist (Korngröße: 5 mm, kugelförmig, 60 A Porenweite; Säulenlänge: 250 mm bei einem Innendurchmesser von 4,6 mm; Fließmittel: n-Hexan/Tetrahydrofuran/Methylenchlorid/Äthanol (90/10/1/1); Fließgeschwindigkeit: 2 ml/Minute; Temperatur:
20°C; UV-Detektion bei 242 nm).
Gefunden: Peak 1(R): Peak 2(S) = 0,75 %: 99,25 %,
ee = 98,5 % (S).

### Beispiel 3

### (S)-2-Äthoxy-4-[N-(1-(2-piperidino-phenyl)-3-methyl-1-butyl)-aminocarbonylmethyl]-benzoesäure-äthylester

Zu einer Lösung von 2,71 g (11 mMol) wasserfreiem (S)-3-Methyl-1-(2-piperidino-phenyl)-1-butylamin (ee = 98,5 %) in 30 ml absolutem Toluol gibt man bei Raumtemperatur 2,77 g (11 mMol) 3-Äthoxy-4-äthoxycarbonyl-phenylessigsäure und rührt, bis Lösung erreicht ist. Dann gibt man 2,38 g (11,55 mMol) N,N'-Dicyclohexyl-carbodiimid zu und rührt bei Raumtemperatur. Nach 24 Stunden setzt man weitere 0,54 g (2,14 mMol) 3-Äthoxy-4-äthoxycarbonyl-phenylessigsäure und 0,48 g (2,33 mMol) N,N'-Dicyclohexyl-carbodiimid zu und rührt über Nacht. Anschließend kühlt man auf eine Innentemperatur von +5°C ab und nutscht vom ausgefallenen Niederschlag ab, den man einmal mit 5 ml Toluol wäscht. Die vereinigten Toluol-Filtrate engt man im Vakuum auf ein Volumen von ca. 10 ml ein. Die so erhaltene Lösung erhitzt man auf dem Dampfbad und versetzt sie portionsweise mit Petroläther (insgesamt 55 ml) bis zur bleibenden Trübung. Man kühlt in Eis ab, wobei Kristallisation erfolgt. Man nutscht ab und trocknet bei 75°C/4 Torr. Das erhaltene Produkt (4,57 g; Schmelzpunkt 111-112°C; ee = 98,9 %) suspendiert man in 50 ml Petroläther. Man erhitzt auf dem Dampfbad und gibt portionsweise soviel Toluol (insgesamt 8 ml) zu, bis Lösung erzielt ist. Dann kühlt man in Eis ab und nutscht vom Kristallisat ab, das man bei 75°C/4 Torr trocknet.
Ausbeute: 3,93 g (74,3 % der Theorie),
Schmelzpunkt: 117-118°C

| | | | |
|---|---|---|---|
| Ber. | C 72,47 | H 8,39 | N 5,83 |
| Gef. | 72,44 | 8,43 | 5,93 |

[a]²⁰_{D} = + 9,4° (c = 1,01 in Methanol)
Enantiomeren-Reinheit: ee = 99,9 ± [HPLC-Methode: siehe Beispiel 2]

### Beispiel 4

### (S)-2-Äthoxy-4-[N-(1-(2-piperidino-phenyl)-3-methyl-1-butyl)-aminocarbonylmethyl]benzoesäure

Man rührt die Lösung von 3,79 g (7,88 mMol) (S)-2-Äthoxy-4-[N-(1-(2-piperidino-phenyl)-3-methyl-1-butyl)-aminocarbonylmethyl]benzoesäure-äthylester (ee = 99,9 %) in 37 ml Äthanol im Bad von 60°C und setzt 10 ml (10 mMol) 1N-Natronlauge zu. Nach 4 Stunden Rühren bei 60°C gibt man in der Wärme 10 ml (10 mMol) 1N-Salzsäure zu und läßt auf Raumtemperatur abkühlen. Nach Animpfen und Stehenlassen über Nacht kühlt man noch für eine Stunde unter Rühren in Eis ab. Man nutscht vom Kristallisat ab und wäscht es zweimal mit je 5 ml Wasser. Anschließend trocknet man bei 75°C bis zuletzt 100°C/4 Torr im Vakuum-Trockenschrank über Phosphorpentoxid.
Ausbeute: 3,13 g (87,7 % der Theorie),
Schmelzpunkt: 130-131°C (hochschmelzende Form)

| | | | |
|---|---|---|---|
| Ber. | C 71,64 | H 8,02 | N 6,19 |
| Gef. | 71,48 | 7,87 | 6,39 |

[a]²⁰_{D} = + 7,45° (c = 1,06 in Methanol)

Die Enantiomeren-Reinheit bestimmt man mittels HPLC auf einer Chiral-Phasen-HPLC-Säule der Firma ChromTech (Schweden) mit einer AGP(α1-acid glycoprotein)-Phase; Innendurchmesser: 4,0 mm; Länge: 100 mm; Teilchendurchmesser: 5 mm; Tempera tur: 20°C; Fließmittel: 0,1%ige wässrige KH₂PO₄-Lösung (=A) + 20 % Acetonitril (=B), Gradientensteigerung binnen 4 Minuten auf 40 % (B); Fließgeschwindigkeit: 1 ml/Minute; UV-Detektion bei 240 nm. Retentionszeit (S)-Enantiomer: 2,7 Minuten; Retentionszeit (R)-Enantiomer: 4,1 Minuten.
Gefunden: (S):(R) = 99,85 %: 0,15 %,
ee = 99,7 % (S).

### Beispiel 5

### (R)(+)-2-Äthoxy-4- [N-(1-(2-piperidino-phenyl)-3-methyl-1-butyl)-aminocarbonylmethyl]benzoesäure x 0,4 H₂O

Hergestellt aus 150 mg (0,312 mMol) (R)(+)-2-Äthoxy-4-[N-(1-(2-piperidino-phenyl)-3-methyl-1-butyl)-aminocarbonylmethyl]-benzoesäure-äthylester [Schmelzpunkt: 122-124°C;
[a]²⁰_{D} = - 8,3° (c = 1 in Methanol)] durch Verseifung mit 1N-Natronlauge in Ethanol analog Beispiel 4.
Ausbeute: 95,8 mg (66,7 % der Theorie),
Schmelzpunkt: 103-105°C (Toluol/Petroläther)

| | | | | |
|---|---|---|---|---|
| Ber. | (x 0,4 H₂O) | C 70,51 | H 8,01 | N 6.09 |
| Gef. | | 70,88 | 7,79 | 5.81 |

- Molpeak M⁺:: Ber.: 452
Gef.: 452
[a]²⁰_{D} = - 6,5° (c = 1 in Methanol)
Enantiomeren-Reinheit: ee = 99,7 % (R) [HPLC-Methode: siehe Beispiel 4].

### Beispiel 6:

### (S)(+)-2-Äthoxy-4-[N-(1-(2-piperidino-phenyl)-3-methyl-1-butyl)-aminocarbonylmethyl]benzoesäure x 0,4 H₂O

Hergestellt aus 89 mg (0,198 mMol) (S)(+)-2-Äthoxy-4-[N-(1-(2-piperidino-phenyl)-3-methyl-1-butyl)-aminocarbonylmethyl]-benzoesäure-äthylester [Schmelzpunkt: 122-124°C;
[a]²⁰_{D} = + 8,3° (c = 1 in Methanol)] durch Verseifung mit 1N-Natronlauge in Äthanol analog Beispiel 4.
Ausbeute: 44,5 mg (48,8 % der Theorie),
Schmelzpunkt: 102-103°C (Toluol/Petroläther)

| | | | |
|---|---|---|---|
| Ber. | (x 0,4 H₂O) | C 70,51 | H 8,01 |
| Gef. | | 70,80 | 8,06 |

[a]²⁰_{D} = + 6,7° (c = 1 in Methanol)
Enantiomeren-Reinheit: ee = 99,6 % (S) [HPLC-Methode: siehe Beispiel 4].

### Beispiel 7

### (S)-2-Äthoxy-4-[N-(1-(2-piperidino-phenyl)-3-methyl-1-butyl)-aminocarbonylmethyl]benzoesäure

Man hydriert 0,26 g (0,47 mMol) (S)-2-Äthoxy-4-[N-(1-(2-piperidino-phenyl)-3-methyl-1-butyl)-aminocarbonylmethyl]-benzoesäure-benzylester (Schmelzpunkt: 91-92°C;
[a]²⁰_{D} = + 9,5°; c = 1,05 in Methanol) in 10 ml Ätha nol an 0,12 g Palladium/Kohle (10%ig) bei 50°C und 5 bar Wasserstoff. Nach 5 Stunden filtriert man den Katalysator über Kieselgur ab und dampft im Vakuum ein. Den Eindampfrückstand kristallisiert man aus Äthanol/Wasser (2/1).
Ausbeute: 0,15 g (70 % der Theorie),.
Schmelzpunkt: 130-131°C

| | | | |
|---|---|---|---|
| Ber. | C 71,64 | H 8,02 | N 6,19 |
| Gef. | 71,76 | 8,12 | 6,05 |

Enantiomeren-Reinheit: ee = 99,6 % [HPLC-Methode: siehe Beispiel 4].

### Beispiel 8

### (S)-2-Äthoxy-4-[N-(1-(2-piperidino-phenyl)-3-methyl-1-butyl)-aminocarbonylmethyl]-benzoesäure

Man erhitzt 102 mg (0,20 mMol) (S)-2-Äthoxy-4-[N-(1-(2-piperidino-phenyl)-3-methyl-1-butyl)-aminocarbonylmethyl]-benzoesäure-tert.butylester (Schmelzpunkt: 122-123°C;
[a]²⁰_{D} = + 8,7° ; c = 1 in Methanol) in 5 ml Benzol zusammen auf einigen Kristallen p-Toluolsulfonsäure-hydrat einen halben Tag auf Rückfluß. Dann ist laut Dünnschichtchromatogramm nach R_{f}-Wert und Massenspektrum das gewünschte Produkt entstanden.
Schmelzpunkt: 129-131°C
- Molpeak M⁺: Ber.: 452
Gef.: 452

### Beispiel 9

### (S)-2-Äthoxy-4-[N-(1-(2-piperidino-phenyl)-3-methyl-1-butyl)-aminocarbonylmethyl]-benzoesäure

Man rührt 200 mg (0,395 mMol) (S)-2-Äthoxy-4-[N-(1-(2-piperidino-phenyl)-3-methyl-1-butyl)-aminocarbonylmethyl]-benzoesäure-tert.butylester (Schmelzpunkt: 122-123°C;
[a]²⁰_{D} = + 8,7°; c = 1 in Methanol) in 2 ml Methylen chlorid zusammen mit 0,45 g (3,95 mMol) Trifluoressigsäure über Nacht bei Raumtemperatur. Man dampft im Vakuum ein und verteilt den Eindampfrückstand zwischen wässriger Natriumhydrogencarbonat-Lösung und Äthylacetat. Man trocknet den organischen Extrakt, filtriert ihn und dampft im Vakuum ein. Den Eindampfrückstand kristallisiert man aus Äthanol/Wasser (2/1). Ausbeute: 115 mg (64,7 % der Theorie),
Schmelzpunkt: 126-128°C

| | | | |
|---|---|---|---|
| Ber. | C 71,64 | H 8,02 | N 6,19 |
| Gef. | 71,39 | 7,91 | 6,06 |

[a]²⁰_{D} = + 6,97° (c = 0,975 in Methanol)

Enantiomeren-Reinheit: ee = 99,8 % [HPLC-Methode: siehe Beispiel 4].

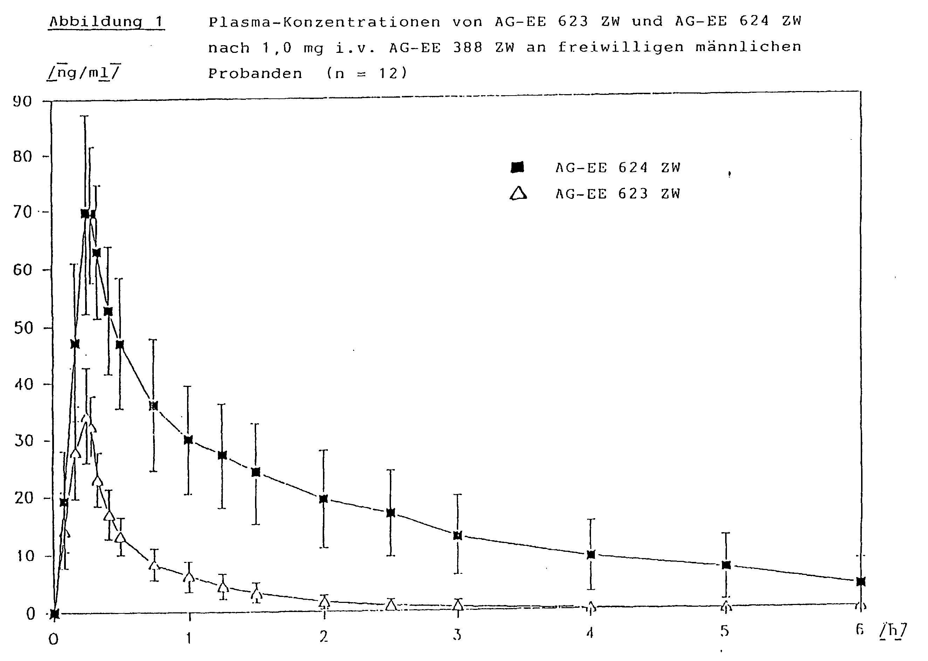

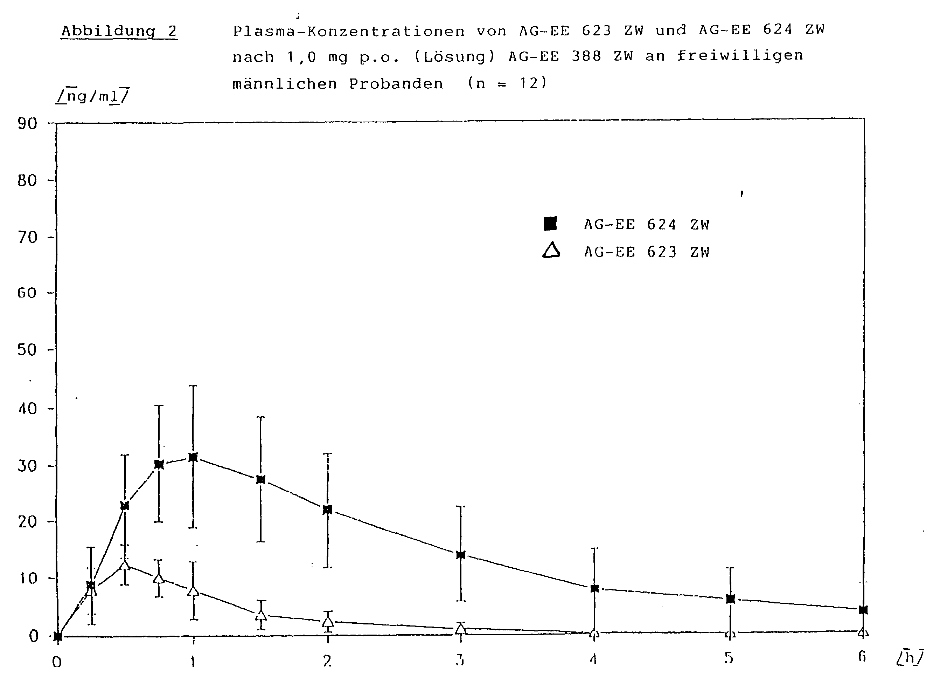

## Patentansprüche

1. Verfahren zur Herstellung von (S)-1-(2-Piperidino-phenyl)-3-methyl-1-butylamin durch Racematspaltung des racemischen 1-(2-Piperidino-phenyl)-3-methyl-1-butylamins mittels fraktionierter Kristallisation der diastereomeren Salze mit einer optisch aktiven Säure und erforderlichenfalls Umkristallistation sowie anschließender Zerlegung der Salze, **dadurch gekennzeichnet, daß** als optisch aktive Säure N-Acetyl-L-glutaminsäure verwendet wird.

2. Verfahren nach Anspruch 1, **dadurch gekennzeichnet, daß**
(a) racemisches 1-(2-Piperidino-phenyl)-3-methyl-1-butylamin und N-Acetyl-L-Glutaminsäure zusammen gelöst werden, das ausgefallene Salz von (S)-1-(2-Piperidino-phenyl)-3-methyl-1-butylamin mit N-Acetyl-L-Glutaminsäure abgetrennt und erforderlichenfalls erneut kristallisiert wird und
(b) das reine (S)-1-(2-Piperidino-phenyl)-3-methyl-1-butylamin aus dem so erhaltenen Salz freigesetzt wird.

3. Verfahren nach Anspruch 2, **dadurch gekennzeichnet, daß**
(a) eine Lösung des racemischen Amins in Aceton mit der Säure versetzt und zum Rückfluß erhitzt wird, wobei bis zur Klärung der Lösung portionsweise Methanol als Lösungsmittel zugegeben wird,
(b) die so erhaltene Lösung abgekühlt und das dabei auskristallisierte Produkt aus Aceton/Methanol im Verhältnis 5:1 umkristallisiert wird.

4. Verwendung des nach dem Verfahren gemäß Anspruch 1 erhaltenen enantiomerenreinen (S)-1-(2-Piperidino-phenyl)-3-methyl-1-butylamins zur Herstellung von (S)-(+)-2-Ethoxy-4-[N-[1-(2-piperidino-phenyl)-3-methyl-1-butyl]amino-carbonylmethyl]benzoesäure, **dadurch gekennzeichnet, daß** das enantiomerenreine (S)-1-(2-Piperidino-phenyl)-3-methyl-1-butylamin mit einer Carbonsäure der allgemeinen Formel 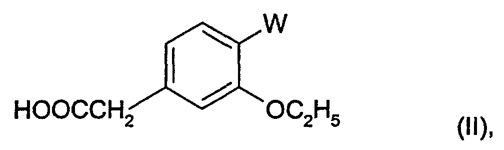 in der
W eine Carboxygruppe oder eine durch einen Schutzrest geschützte Carboxygruppe darstellt,
oder mit deren gegebenenfalls im Reaktionsgemisch hergestellten reaktionsfähigen Derivaten umgesetzt und erforderlichenfalls anschließend der Schutzrest abgespalten wird.

5. Verwendung von (S)-1-(2-Piperidino-phenyl)-3-methyl-1-butylamin gemäß Anspruch 4, **dadurch gekennzeichnet, daß** die Carbonsäure der Formel (II) 3-Ethoxy-4-ethoxycarbonyl-phenylessigsäure ist.

## Claims

1. Process for preparing (S)-1-(2-piperidino-phenyl)-3-methyl-1-butylamine by splitting the racemates of racemic 1-(2-piperidino-phenyl)-3-methyl-1-butylamine via fractional crystallisation of the diastereomeric salts with an optically active acid and if necessary recrystallisation and subsequent decomposition of the salts, **characterised in that** N-acetyl-L-glutamic acid is used as the optically active acid.

2. Process according to claim 1, **characterised in that**
(a) racemic 1-(2-piperidino-phenyl)-3-methyl-1-butylamine and N-acetyl-L-glutamic acid are dissolved together, the precipitated salt of (S)-1-(2-piperidino-phenyl)-3-methyl-1-butylamine with N-acetyl-L-glutamic acid is separated off and if necessary crystallised again, and
(b) the pure (S)-1-(2-piperidino-phenyl)-3-methyl-1-butylamine is liberated from the salt thus obtained.

3. Process according to claim 2, **characterised in that**
(a) a solution of the racemic amine in acetone is combined with the acid and refluxed, while methanol as solvent is added batchwise until the solution becomes clear,
(b) the resulting solution is cooled and the product which has crystallised out is recrystallised from acetone/methanol in the ratio 5:1.

4. Use of the enantiomerically pure (S)-1-(2-piperidino-phenyl)-3-methyl-1-butylamine obtained by the process according to claim 1, for preparing (S)-(+)-2-ethoxy-4-[N-[1-(2-piperidino-phenyl)-3-methyl-1-butyl]amino-carbonylmethyl]benzoic acid, **characterised in that** the enantiomerically pure (S)-1-(2-piperidinophenyl)-3-methyl-1-butylamine is reacted with a carboxylic acid of general formula 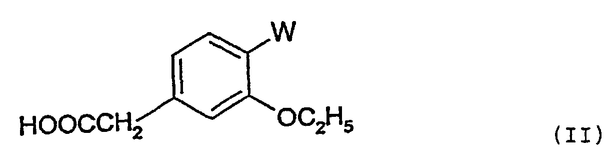 wherein
W denotes a carboxy group or a carboxy group protected by a protecting group,
or with the reactive derivatives thereof optionally prepared in the reaction mixture and subsequently, if desired, the protecting group is cleaved.

5. Use of (S)-1-(2-piperidino-phenyl)-3-methyl-1-butylamine according to claim 4, **characterised in that** the carboxylic acid of formula (II) is 3-ethoxy-4-ethoxycarbonyl-phenylacetic acid.

## Revendications

1. Méthode de préparation de la (S)-1-(2-pipéridinophényl)-3-méthyl-1-butylamine par résolution de l'amine racémique 1-(2-pipéridinophényl)-3-méthyl-1-butylamine au moyen d'une cristallisation fractionnée des sels diastéréomères avec un acide optiquement actif et, si nécessaire, d'une recristallisation suivie d'une décomposition du sel, **caractérisée en ce que** l'acide optiquement actif utilisé est l'acide N-acétyl-L-glutaminique.

2. Méthode selon la revendication 1 **caractérisée en ce que**
(a) la 1-(2-pipéridinophényl)-3-méthyl-1-butylamine racémique et l'acide N-acétyl-L-glutaminique sont mis en solution ensemble, le sel précipité de (S)-1-(2-pipéridinophényl)-3-méthyl-1-butylamine et d'acide N-acétyl-L-glutaminique étant séparé et, si nécessaire, recristallisé et
(b) la (S)-1-(2-pipéridinophényl)-3-méthyl-1-butylamine pure est libérée du sel ainsi obtenu

3. Méthode selon la revendication 2 **caractérisée en ce que**
(a) une solution de l'amine racémique dans l'acétone est mélangée avec l'acide et chauffée à reflux, du méthanol servant de solvant étant ajouté par fractions jusqu'à éclaircissement de la solution,
(b) la solution ainsi obtenue est refroidie et le produit cristallisé est recristallisé dans l'acétone/méthano dans une proportion de 5:1.

4. Utilisation de l'amine (S)-1-(2-pipéridinophényl)-3-méthyl-1-butylamine optiquement pure obtenue selon la méthode de la revendication 1 pour synthétiser l'acide (S)-(+)-2-éthoxy-4-[N- [1-(2-pipéridinophényl)-3-méthyl-1-butyl]aminocarbonylméthyl]-benzoïque **caractérisée en ce que** la (S)-1-(2-pipéridinophényl)-3-méthyl-1-butylamine optiquement pure réagit avec un acide carboxylique de formule générale 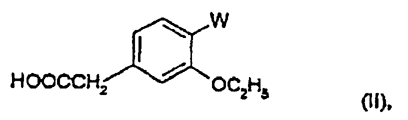 dans laquelle
W représente un groupement carboxyle ou un groupement carboxyle protégé par un radical protecteur,
ou avec ses dérivés réactifs éventuellement formés dans le mélange réactionnel, avec séparation ultérieure éventuelle du radical protecteur.

5. Utilisation de la (S)-1-(2-pipéridinophényl)-3-méthyl-1-butylamine selon la revendication 4 **caractérisée en ce que** l'acide carboxylique de formule (II) est l'acide 3-éthoxy-4-éthoxycarbonylphénylacétique.
